# EUROPÄISCHE PATENTSCHRIFT

(11) **EP 2 129 289 B1**
(45) Veröffentlichungstag und Bekanntmachung des Hinweises auf die Patenterteilung: **09.07.2014**
(21) Anmeldenummer: 08716244.2
(22) Anmeldetag: 05.03.2008
(51) Int. Cl.: A61B 5/15

(54) **ANALYSESYSTEM ZUR BESTIMMUNG EINES ANALYTEN IN EINER KÖRPERFLÜSSIGKEIT UND DISPOSIBLES INTEGRIERTES PROBENGEWINNUNGS- UND ANALYSEELEMENT**
ANALYTICAL SYSTEM FOR DETERMINING AN ANALYTE IN A BODY FLUID AND DISPOSABLE INTEGRATED SAMPLE COLLECTION AND ANALYTICAL ELEMENT
SYSTÈME D'ANALYSE POUR LA DÉTERMINATION D'UN ANALYTE DANS UN LIQUIDE CORPOREL, ET ÉLÉMENT DE PRÉLÈVEMENT D'ÉCHANTILLON ET D'ANALYSE INTÉGRÉ JETABLE

(30) Priorität: 14.03.2007 EP 07005222
(43) Veröffentlichungstag der Anmeldung: 09.12.2009
(73) Patentinhaber: Roche Diagnostics GmbH, 68305 Mannheim (DE); F. Hoffmann-La Roche AG, 4070 Basel (CH)
(72) Erfinder: LIST, Hans, 64754 Hesseneck-Kailbach (DE); ZIMMER, Volker, 54497 Morbach (DE); KEIL, Michael, 67071 Ludwigshafen (DE)
(74) Vertreter: Durm & Partner
(86) Internationale Anmeldenummer: PCT/EP2008/001726
(87) Internationale Veröffentlichungsnummer: WO 2008/110287

(56) Entgegenhaltungen:
- WO-A-2006/092281
- WO-A-2007/025713

## Beschreibung

Die vorliegende Erfindung betrifft ein Analysesystem zur Bestimmung eines Analyten in einer Körperflüssigkeit mit einem disposiblen integrierten Probengewinnungs- und Analyseelement und mit einem wiederverwendbaren Analysegerät. Das disposible Probengewinnungs- und Analyseelement schließt ein Stechelement mit einer Spitze zum Erzeugen einer Einstichwunde in einem Körperteil und einen flachen Teststreifen mit einer Probenaufnahmezone zur Aufnahme einer aus der Einstichwunde austretenden Körperflüssigkeitsprobe ein. Das Analysegerät umfasst eine Kopplungseinheit zum Koppeln eines Probengewinnungs- und Analyseelementes mit einem Antrieb, durch den eine Stechbewegung des Stechelementes angetrieben wird, bei dem es auf einem Einstichweg während der Vortriebsphase in Einstichrichtung und nach Erreichen des Umkehrpunktes der Stechbewegung während der Rückführungsphase entgegen der Einstichrichtung bewegt wird. Das Analysegerät umfasst auch eine Mess- und Auswerteeinheit zur Messung einer für die Bestimmung des Analyten charakteristischen Messgröße und zur Ermittlung eines gewünschten Analyseergebnisses auf der Grundlage der Messung. Dabei ist die Probenaufnahmezone des Teststreifens an einer seiner Flachseiten, die eine Probenkontaktseite bildet, angeordnet. Das Stechelement ist parallel zu dem Teststreifen angeordnet und während mindestens eines Teils der Einstichbewegung relativ zu dem Teststreifen auf einem Bewegungsweg bewegbar.

Für Diagnosezwecke werden geringe Mengen von Körperflüssigkeiten, wie beispielsweise Blut, aus einem Körperteil entnommen. Hierzu werden in der Regel Stechgeräte mit Lanzetten verwendet, mit denen eine Wunde in dem Körperteil, beispielsweise in dem Finger oder in dem Ohrläppchen, erzeugt wird. Die Stechsysteme sind derart ausgebildet, dass sie auch von Laien verwendet werden können.

Allerdings bedarf es bei der Bestimmung eines Analyten in der Körperflüssigkeit eines Vorgehens in mehreren Schritten. Zuerst muss mit einem Stechsystem, das ein Stechgerät und eine Lanzette umfasst, eine Wunde in dem Körperteil erzeugt werden, aus der dann eine Körperflüssigkeit, wie z.B. Blut, austritt. In einem weiteren Schritt muss die Flüssigkeit von einem Testelement aufgenommen und einem Analysesystem zugeführt werden, mit dem der gesuchte Analyt in der Körperflüssigkeit bestimmt wird.

Dieses Vorgehen ist aufwendig, insbesondere für Diabetiker, die den Glucosegehalt im Blut mehrfach am Tag bestimmen müssen. Deshalb wird neben einem möglichst schmerzfreien Einstich auch ein erhöhter Bedienkomfort gefordert.

Um dem gewünschten Bedienkomfort einen Schritt näher zu kommen, werden im Stand der Technik integrierte Analysesysteme vorgeschlagen, die neben einem Stechgerät auch eine Analyseeinheit umfassen. Aus der WO 2006/027101 A1 ist ein Analysesystem bekannt, bei dem nach der Erzeugung der Wunde im Körperteil das Stechgerät von einer Öffnung des Analysesystems wegbewegt und in einem zweiten Schritt eine Analyseeinheit an die Öffnung des Systems hinbewegt wird, so dass aus der Wunde austretendes Blut von der Analyseeinheit aufgenommen werden kann.

Um die Handhabbarkeit und den Komfort der Analysesysterne zu verbessern, wurden Analysesysteme entwickelt, die Testelemente bzw. Testsensoren mit einer integrierten Lanzette vorschlagen. Ein derartiges System, bei dem die Lanzette unterhalb des Testfelds eines Testelements angeordnet ist, ist beispielsweise aus der WO2007/025713 bekannt. Die EP 1 894 526 A1 zeigt eine Lanzette, die von einer Schutzhülle umschlossen ist, wobei die Schutzhülle ein Testfeld für die Analyse hat. Der Teil der Schutzhülle mit dem Testfeld wird vor dem Gebrauch der Lanzette manuell soweit von der Lanzette weggebogen, dass die Lanzette aus der Schutzhülle heraustreten kann, ohne das Testfeld auf dem Bewegungsweg zu zerkratzen.

Beispielsweise ist aus der WO2006/092281 ein Analysesystem mit einem Testsensor mit integrierter Lanzette bekannt, bei dem ein Analyt in einer Körperflüssigkeit durch eine elektrochemische Messung bestimmt wird. Der disposible Testsensor umfasst einen Teststreifen, an dessen Oberseite ein Kapillarkanal vorgesehen ist, der zur Weiterleitung der aufgenommenen Körperflüssigkeit an Testelektroden dient, die ebenfalls an der Oberseite des Teststreifens angeordnet sind, um einen Analyten in der Körperflüssigkeit zu bestimmen. An der Unterseite des Teststreifens ist eine Lanzette angeordnet, die relativ zu dem Teststreifen beweglich gelagert ist. Die Lanzette wird von einer sterilen Hülle umschlossen, aus der sie vor dem Einstich in das Körperteil heraustritt.

Zur Probengewinnung wird der Testsensor in die Nähe der Öffnung des Analysesystems geführt. Dann wird die Lanzette in Einstichrichtung nach vorne bewegt, bis sie aus der Öffnung des Aufnahmesystems hinaustritt und in einem an der Öffnung anliegenden Körperteil eine Wunde erzeugt. Nach dem Einstich wird die Lanzette wieder zurückgezogen, bis sie wieder in ihrer sterilen Schutzhülle positioniert wird. Das aus der Einstichwunde austretende Blut bzw. die austretende Körperflüssigkeit wird von dem Kapillarkanal an der Oberseite des Teststreifens angesaugt und gelangt schließlich an die Elektroden, so dass auf elektrochemischem Wege ein Analyt in der Körperflüssigkeit bestimmt werden kann. Nachteilig an einem derartigen System ist, dass zwei getrennte Handhabungsschritte erforderlich sind, um einerseits den Stich in die Haut und andererseits die Übertragung einer hinreichend großen Blutprobe auf das Testelement zu gewährleisten.

Dies kann manuell erfolgen, wobei nach Ausführung des Stichs das Analysesystem von dem Körperteil entfernt und das Körperteil anschließend "gemolken" wird, um den Blutaustritt aus der Wunde zu fördern. Sobald eine genügend große Blutmenge aus der Wunde ausgetreten ist, wird das Analysesystem wieder manuell an die Wunde hingeführt, um die Probe in den Kapillarkanal anzusaugen. Dies bedingt eine umständliche Handhabung durch den Patienten und fällt insbesondere älteren Menschen schwer, die von Diabetes häufig betroffen sind.

Alternativ zu der manuellen Vorgehensweise und der mehrere Schritte umfassenden Bedienung kann das "Melken" durch das Analysesystem selbst mechanisiert werden. Dazu wurden Systeme mit einem sogenannten Fingerkonus vorgeschlagen. Nach dem Stechvorgang wird durch Druck auf den Konus die Expression einer Flüssigkeitsprobe bewirkt. Hierbei wird also die manuelle Handhabung durch eine entsprechende Gerätefunktion ersetzt, was jedoch einen erheblichen konstruktiven Aufwand bedeutet und die Geräte verteuert.

Als dritte, jedoch eher theoretische Möglichkeit, die auch der Forderung nach einem möglichst schmerzarmen Stich entgegensteht, kann die Lanzette so tief in das Körperteil eingestochen werden, dass ein ausreichend großer Blutstropfens ohne zusätzliche Maßnahmen wie manuelles oder mechanisches Melken austritt. Der mit dem tiefen Einstich verbundene Schmerz ist jedoch so groß, dass ein derartiges System für die Praxis ungeeignet ist.

Es ist somit Aufgabe der vorliegenden Erfindung, ein Analysesystem vorzuschlagen, das sich durch einen hohen Komfort für den Benutzer auszeichnet, insbesondere dadurch, dass er das Analysesystem nur einmal an das Körperteil anhalten muss, um den gewünschten Analyten zu bestimmen. Andererseits soll das System leicht zu bedienen sein und auf einer einfachen und kostengünstigen Konstruktion beruhen.

Gelöst wird die vorliegende Aufgabe durch ein disposibles Probengewinnurigs- und Analyseelement mit den Merkmalen des Anspruchs 1 und durch ein System nach Anspruch 7 mit einem Probengewinnungs- und Analyseelement nach Anspruch 1.

Das erfindungsgemäße Analysesystem zur Bestimmung eines Analyten in einer Körperflüssigkeit umfasst ein disposibles integriertes Probengewinnungs- und Analyseelement, das in Fachkreisen auch als "Dispo" bezeichnet wird, und ein wiederverwendbares Analysegerät. Das disposible integrierte Probengewinnungs- und Analyseelement hat ein Stechelement mit einer Spitze zum Erzeugen einer Einstichwunde in einem Körperteil und einen flachen Teststreifen mit einer Probenaufnahmezone, in die eine Körperflüssigkeitsprobe zur Durchführung einer Analyse übertragen wird. Das wiederverwendbare Analysegerät schließt eine Kopplungseinheit ein, die derart ausgebildet ist, um ein disposibles integriertes Probengewinnungs- und Analyseelement (Dispo) mit einem Antrieb zu koppeln, durch den eine Stechbewegung des Stechelements des Dispos angetrieben wird. Bei der Stechbewegung wird das Stechelement auf einem Einstichweg während einer Vortriebsphase in Einstichrichtung und nach Erreichen eines Umkehrpunktes der Stechbewegung während einer Rückführungsphase entgegen der Einstichrichtung bewegt. Das Analysegerät schließt darüber hinaus eine Mess- und Auswerteeinheit ein, die zur Messung einer für die Bestimmung des Analyten charakteristischen Messgröße ausgebildet ist, und mittels der auf Grundlage der Messung eine Ermittlung des erwünschten Analyseergebnisses stattfindet.

Nachfolgend wird ohne Einschränkung der Allgemeinheit, auf Blut als Beispiel einer Körperflüssigkeit Bezug genommen. Selbstverständlich ist es mit dem erfindungsgemäßen System auch möglich, Analyten in anderen Körperflüssigkeiten zu bestimmen.

Die Probenaufnahmezone des Teststreifens des Dispo ist an einer der Flachseiten des Teststreifens angeordnet. Diese Flachseite bildet eine Probenkontaktseite. Das Stechelement ist parallel zu dem Teststreifen angeordnet und ist während mindestens eines Teils der Einstichbewegung relativ zu den Teststreifen auf einem Bewegungsweg bewegbar. Das Stechelement ist zu der Probenkontaktseite des Teststreifens des Dispos benachbart, also an der Flachseite angeordnet, an der die Probenaufnahmezone liegt. Es weist einen Kapillarkanal auf, der einen Probeneinlass und einen Probenauslass hat. Durch den Probeneinlass kann die Körperflüssigkeit nach dem Einstich in den Kapillarkanal eintreten; durch den Probenauslass (sample outlet) kann sie aus dem Kanal wieder heraustreten.

Die durch den Probenauslass aus dem Kapillarkanal austretende Körperflüssigkeit kann zu der Probenaufnahmezone übertragen werden, wenn der Probenauslass der Probenaufnahmezone des Teststreifens benachbart ist. Diese Position, in der das Stechelement relativ zu dem Teststreifen so positioniert ist, dass der Probenauslass an die Probenaufnahmezone angenähert ist, wird Probenübertragungsposition (sample transfer position) genannt. Der Bewegungsweg des Stechelements schließt diese Position folglich ein.

Das erfindungsgemäße Analysesystem sowie auch das disposible integrierte Probengewinnungs- und Analyseelement zeichnen sich durch einen einfachen Aufbau aus. Wenn das Stechelement mit seiner Spitze bei der Einstichbewegung in das Körperteil eindringt und eine Wunde erzeugt, kann Körperflüssigkeit durch den Probeneinlass in den Kapillar-kanal eindringen. Die Körperflüssigkeit wird also gezielt schon beim Einstich in den Kapillarkanal geführt Auch während der Rückführungsphase der Stechbewegung des Stechelements dringt weiter Körperflüssigkeit in den Kapillarkanal ein und wird durch diesen zum Probenauslass geführt.

Der Austritt der Körperflüssigkeit aus dem Kapillarkanal findet erfindungsgemäß in der Probenübertragungsposition statt. Wenn sich das Stechelement in dieser Position befindet, ist wenigstens ein Teil des Probenauslasses derart an der Probenaufnahmezone lokalisiert, dass die Körperflüssigkeit Vom Kapillarkahal auf die Probenaufnahmezone übertreten kann. Diese Übergabestelle ist also eine definierte Position, so dass auf einfache Weise sichergestellt wird, dass die Körperflüssigkeit zur Probenaufnahmezone gelangt. Vorteilhaft erweist sich, dass der Einstich und die Aufnahme der Körperflüssigkeit mit dem gleichen Element stattfindet, nämlich mit dem Stechelement.

Für den Benutzer des Analysegeräts ergibt sich dadurch der Vorteil, dass es sehr einfach zu bedienen ist. Er muss das Gerät zur vollständigen Durchführung einer Analyse nur einmal an sein Körperteil, vorzugsweise an die Fingerkuppe, ansetzen. Weitere Handhabungsschritte des Benutzers, beispielsweise Abnehmen des Gerätes nach dem Einstich, mechanisches Melken und Wiederansetzen des Gerätes an die Einstichwunde zur Aufnahme der austretenden Körperflüssigkeit in einen Kapillarkanal, entfallen. Somit ist ein echtes "one-stop-handling" für den Benutzer möglich. Hierdurch ist eine hohe Akzeptanz bei den Benutzern gegeben. Das erfindungsgemäße Analysesystem kommt gerade älteren Menschen entgegen, deren feinmotorische Fähigkeiten geschwächt.

Die zuverlässige Übertragung der Körperflüssigkeit zu der Probenaufnahmezone wird dadurch unterstützt, dass die Bewegung des Stechelementes auf der Seite des Teststreifens stattfindet, die als Probenkontaktseite bezeichnet wird und die Probenaufnahmezone einschließt. Damit ist eine einfache und zuverlässige Übertragung möglich.

In einer vorteilhaften Ausführungsform wird die Probengewinnung dadurch verbessert, dass sich an die Stechbewegung des Stechelements eine Sammelbewegung anschließt, während der eine Körperflüssigkeitsprobe in den Kapillarkanal des Stechgeräts aufgenommen wird. Die Bewegungsgeschwindigkeit des Stechelements ist während der Sammetbewegung geringer als während der Stechbewegung. Auch die Relativbewegung des Stechelements relativ zu dem Teststreifen sollte während der Sammelbewegung im Vergleich zur Stechbewegung langsamer sein. In einer besonders bevorzugten Ausführungsform werden beide synchron so bewegt, dass ihre Relativposition erhalten bleibt. In diesem Fall findet während der Sammelbewegung keine Relativbewegung zwischen Stechelement und Teststreifen statt.

Optional führt das Stechelement neben der Stechbewegung und der Sammelbewegung auch eine Transferbewegung durch, in der das Stechelement in die Probenübertragungsposition bewegt wird, in der die Übergabe der Körperflüssigkeit aus dem Kapillarkanal zur Probenaufnahmezone stattfindet. Als Bewegungsweg des Stechelements wird der Weg aller Bewegungen des Stechelementes bezeichnet, einschließlich der Stechbewegung der Sammelbewegung und der Transferbewegung.

In einer bevorzugten Ausführungsform des Analysesystems umfasst das Analysegerät eine Kopplungseinheit mit zwei Kopplungsmechanismen. Der erste Kopplungsmechanismus ist zur Kopplung mit dem Stechelement ausgebildet, der zweite Kopplungsmechanismus zur Kopplung mit dem Teststreifen. Das Testelement und der Teststreifen können dadurch unabhängig voneinander bewegt werden, wobei beide Komponenten gleichzeitig mit gleichen oder unterschiedlichen Geschwindigkeiten bewegt werden können. Die Bewegungen können beispielsweise relativ zu einem Gehäuse des Stechgeräts erfolgen. Eine derartige Kopplungseinheit wird vorzugsweise dann eingesetzt, wenn das Stechelement die erläuterte Sammelbewegung ausführen soll. Alternativ zu einer Kopplungseinheit mit zwei Kopplungsmechanismen kann beispielsweise der Teststreifen in dem Gehäuse des Analysegeräts fixiert sein. Dies ist bei einfacheren Ausführungsformen möglich, beispielsweise wenn der Teststreifen zur Probenaufnahme nicht bewegt werden muss.

Bevorzugt umfasst das Analysesystem ein Stapelmagazin, das auswechselbar sein kann und mehrere Dispos (Probengewinnungs- und Analyseelemente) aufnimmt. Das Stapelmagazin kann beispielsweise in einer Halterung des Analysegeräts aufgenommen werden, so dass mehrere Dispos in dem Gerät zur Verfügung stehen. Sobald alle Dispos eines Stapelmagazins verbraucht sind, wird das Magazin ausgewechselt, indem es aus der Halterung gelöst und durch ein neues mit unbenutzten Dispos bestücktes Magazin ersetzt wird. Optional kann das Stapelmagazin auch eine Aufnahme für die benutzten und verbrauchten Probengewinnungs- und Analyseelemente vorsehen.

Die Dispos werden mittels einer Transporteinrichtung einzeln aus dem Stapelmagazin entnommen und können in eine Kopplungsposition bewegt werden. In dieser Kopplungsposition können sie von der Kopplungseinheit angekoppelt werden, um beispielsweise in einem weiteren Schritt in eine Arbeitsposition zu gelangen, in der sie zur Benutzung bereitstehen. Durch eine geeignete Transporteinrichtung und eine angepasste Kopplungseinheit kann die Zuführung der unbenutzten Dispos automatisiert werden. Ebenso kann die Transporteinrichtung geeignet sein, um benutzte Dispos nach ihrer Verwendung erneut zu magazinieren, beispielsweise in einem dafür vorgesehenen Bereich innerhalb des Stapelmagazins.

Die Magazinierung wird dadurch erleichtert, dass der Teststreifen des Dispos flach ausgebildet ist. Bevorzugt ist auch das Stechelement relativ flach, so dass das gesamte Dispo im Vergleich zu seiner Länge und Breite eine sehr geringe Dicke hat. Das Dispo lässt sich dann besonders gut und leicht stapeln.

Optional kann das Stapelmagazin und/oder das Analysegerät mit einer Anzeige versehen sein, die entweder die Anzahl der noch unbenutzten oder der bereits benutzten Dispos anzeigt. Die Anzeige kann in Form von Zahlen oder sonstigen Markierungen oder Indikatoren erfolgen, wie sie im Stand der Technik bekannt sind. Gleichfalls sind im Stand der Technik verschiedene Stapelmagazine bekannt, die sich zum Einsatz in dem erfindungsgemäßen Analysesystem eignen, z.B. aus der EP 0974061 oder der US 6,827,899.

Die Erfindung wird nachfolgend anhand von in den Figuren dargestellten bevorzugten Ausführungsformen näher erläutert. Die dort dargestellten Besonderheiten können einzeln oder in Kombination verwendet werden, um bevorzugte Ausgestaltungen der Erfindung zu schaffen. Es zeigen:
- Fig. 1: ein Analysesystem umfassend ein Analysegerät und ein integriertes Probengewinhungs und Analyseelement;
- Fig. 2a bis d: eine Explosionszeichnung des Probengewinhungs- und Analyseelements aus Figur 1;
- Fig. 3a,b: eine alternative Ausführungsform eines Probengewinnungs- und Analyseelementes;
- Fig. 4a bis 4h: eine schematische Schnittdarstellung eines Teils des Stechsystems nach Figur 1 in acht Benutzungspositionen;
- Fig. 5: eine alternative Ausführungsform eines Probengewinnungs- und Analyseelements;
- Fig. 6: eine weitere Ausführungsform eines Analysegerätes und
- Fig. 7: eine Detailansicht des vorderen Endes eines Probengewinnungs- und Analyseelements.

Die im Weiteren verwendeten Ortsangaben "vorne" und "hinten" beziehen sich auf die Einstichrichtung, in der das Stechelement zur Erzeugung einer Wunde in einem Körperteil bewegt wird. So ist das vordere Ende des Stechelements das Ende, das bei Bewegung des Stechelements in Einstichrichtung zu einer Gehäuseöffnung benachbart ist, an der ein Körperteil zum Erzeugen einer Wunde anliegen kann.

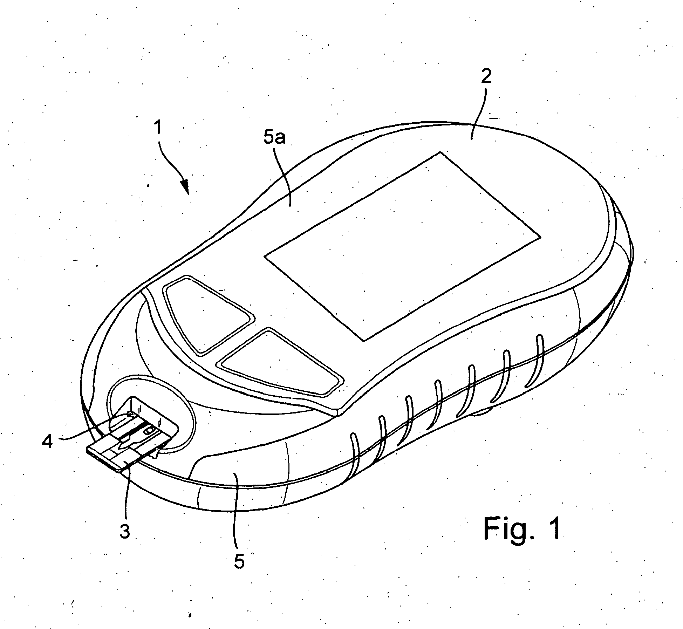

Figur 1 zeigt ein Analysesystem 1 umfassend ein Analysegerät 2'und ein disposibles integriertes Probengewinnungs- und Analyseelement, das als Dispo 3 bezeichnet wird. Das Dispo 3 ragt aus einer Öffnung 4 eines Gehäuses 5 des Analysengerätes 2 teilweise heraus. Bedienelemente zur Bedienung und Steuerung des Analysegerätes 2 sind auf der Oberseite 5a des Gehäuses 5 angeordnet.

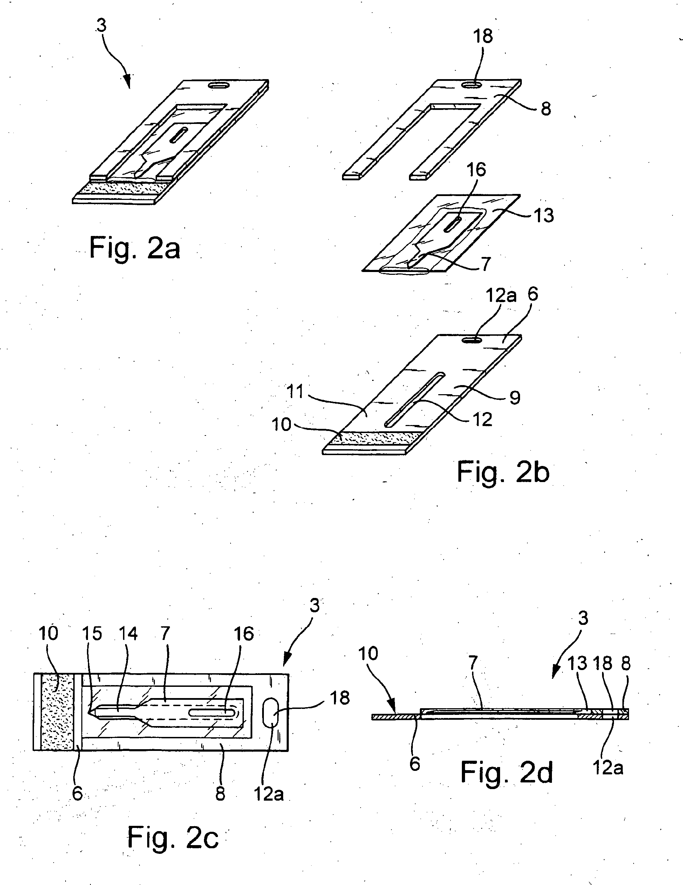

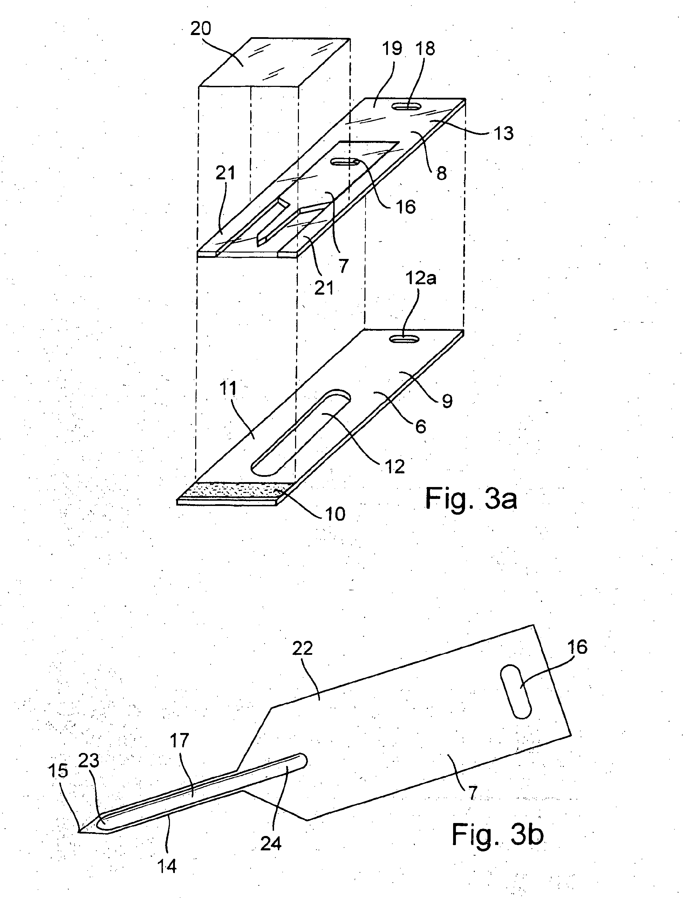

Die Figuren 2a bis d und 3 zeigen verschiedene Ausführungsformen eines Dispos 3. In einer ersten Ausführungsform schließt das Dispo 3 einen länglichen Teststreifen 6, ein Stechelement 7 und ein Deckteil 8 ein. Der Teststreifen 6, dessen Länge vorzugsweise drei bis vier mal größer ist als seine Breite, weist an einer seiner beiden Flachseiten 9 eine Probenaufnahmezone 10 auf. Diese Flachseite 9 wird als Probenkontaktseite 11 bezeichnet. Die Probenaufnahmezone 10 ist vorzugsweise im vorderen Bereich des Teststreifens 6 angeordnet. Als vorderer Bereich ist der Teil des Teststreifens 6 bezeichnet, der dem in Einstichrichtung vorderen Ende des Teststreifens benachbart ist. Dieser Bereich kann direkt am vorderen Ende des Teststreifens beginnen; er kann jedoch auch von der vorderen Kante beabstandet sein. Jedenfalls ist die Probenaufnahmezone 10 näher an dem vorderen Ende des Teststreifens als an dem hinteren Ende angeordnet.

Der Teststreifen weist etwa mittig eine Längsnut 12 auf, die sich in Längsrichtung erstreckt. Die Längsnut 12 dient als Durchgriff eines Kopplungsmechanismusses, der das Stechelement 7 bewegt.

Im Bereich des fernen Endes des Teststreifens 6 ist eine Bohrung 12a angeordnet, die vorzugsweise als Langloch ausgebildet ist und sich quer zur Einstichrichtung im Teststreifen 6 erstreckt. Diese Bohrung 12a dient zur Ankopplung eines Kopplungsmechanismus an den Teststreifen 6, um den Teststreifen 6 im Analysegerät 2 zu bewegen und/oder zu positionieren bzw. zu fixieren.

Das Stechelement 7 ist von einer Schutzhülle 13, beispielsweise einer dünnen Siegelfolie, umschlossen. Die Schutzhülle 13 besteht bevorzugt aus einer Folie, die leicht reissbar und leicht weiterreissbar ist, so dass vor oder zu Beginn der Stechbewegung das Stechelement 7 mit seiner Spitze 15 die Schutzhülle 13 durchstechen kann, um aus ihr herauszutreten. Beim Herstellungsprozess des Dispo 3 kann das Stechelement 7 separat von der Schutzhülle 13 hermetisch eingeschlossen und anschließend sterilisiert werden, so dass es keimfrei gelagert werden kann.

Das vorzugsweise flache Stechelement 7 hat an seinem vorderen Ende ein Nadelelement 14 mit einer Spitze 15, die während der Stechbewegung beim Auftreffen auf die Haut eines Körperteils eine Einstichwunde in dem Körperteil erzeugt. Das Stechelement 7 hat eine Kopplungsausnehmung 16, in die ein Kopplungselement zum Ankoppeln an eine Antriebseinheit eingreifen kann. Die Kopplungsausnehmung 16 ist vorzugsweise ein Langloch.

Das Nadelelement 14 des Stechelements 7 hat einen Kapillarkanal 17 an seiner Unterseite. In dieser bevorzugten Ausführungsform ist der Kapillarkanal 17 zur Probenaufnahmezone 10 des Teststreifens 6 hin orientiert.

Das Dispo 3 ist sandwichartig zusammengebaut, wobei das Stechelement 7 mit der Schutzhülle 13 zwischen dem Deckteil 8 und dem Teststreifen 6 angeordnet ist. Das Deckteil 8 ist in Einstichrichtung offen, damit ein von der Oberseite (Oberschicht) an das Stechelement 7 angekoppeltes Kopplungselement auf dem Bewegungsweg gemeinsam mit dem Stechelement 7 in Einstichrichtung bewegt werden kann. Das Deckteil 8 liegt oben auf der Schutzhülle 13 auf dem Teststreifen 6, um die Stapelbarkeit des Dispos 3 zu verbessern. Das Stechelement 7 wird jedoch nicht gehalten, so dass es sich in Längsrichtung des Dispos 3 bewegen kann. Da das Deckteil 8 das Stechelement 7 nicht berührt, findet keine Führung des Stechelements 7 statt. Das U-förmige Deckteil 8 weist eine Bohrung 18 in seiner Basis auf. Sie korrespondiert mit der Bohrung 12a, so dass das Dispo 3 von einer Kopplungseinheit, die sich durch die Bohrungen 12a,18 erstreckt, gehalten und bewegt werden kann.

In einer alternativen Ausführungsform des Dispos nach Figur 3 wird die Schutzhülle und das Deckteil aus einer konturierten Grundfolie 19 und einer Deckfolie 20 gebildet. Die Grundfolie 19 wird aus einem Boden gebildet, auf dem ein U-förmiges Distanzteil aus Folie aufliegt. Der Boden und das Distanzteil sind einstückig miteinander verbunden. Das Stechelement 7 ist zwischen den beiden U-Schenkel des Distanzteils angeordnet, die Abstandshalter 21 bilden. Vorzugsweise ist das Stechelement also wenigstens auf seinen Längsseiten von Abstandhaltern 21 umgeben, deren Dicke mindestens so groß ist, wie die Dicke des Stechelements 7. Als Dicke wird dabei die Dimension senkrecht zur Probenkontaktseite 11 des Teststreifens 6 definiert. Hierdurch wird beim Zusammenfügen der Grundfolie 19 und der Deckfolie 20 eine Schutzhülle 13 mit einem Hohlraum gebildet, in dem das Stechelement 7 beweglich ist. Bevorzugt sind die Grundfolie 19 und die Deckfolie 20 einstückig ausgebildet, wobei der als Deckfolie 20 dienende vordere Teil der Grundfolie 19 so umgeschlagen wird, dass er auf der Grundfolie 20 liegt und die Umschlagkante an der in Einstichrichtung vorderen Stirnseite angeordnet ist. Die beiden Folien können dann an den drei verbleibenden offenen Seiten miteinander verklebt werden. Das Stechelement 7 tritt auf seinem Einstichweg durch Umschlagkante hindurch, so dass das Stechelement durch keine der Klebeseiten austreten muss.

Das Stechelement 7 ist in dieser Ausführungsform von der Probenkontaktseite 11 des Teststreifens 6 durch die Dicke des Bodens der Grundfolie 19 beabstandet. Das bevorzugt flach ausgebildete Stechelement 7 hat eine in Figur 3a dargestellte Unterseite 22, die zur Probenkontaktseite 11 hingewandt ist. Die Unterseite 22 ist bevorzugt von der Probenaufnahmezone 10 derart beabstandet, dass das Stechelement 7 wenigstens während der Vortriebsphase der Stechbewegung die Probenaufnahmezone 10 nicht berührt. Der senkrecht zur Flachseite 9 des Stechelements 7 definierte Abstand zwischen der Unterseite 22 und der Probenaufnahmezone 10 muss derart sein, dass auch dann, wenn sich das Stechelement 7 während der Stechbewegung direkt oberhalb der Probenaufnahmezone befindet, ein Zwischenraum besteht. Dadurch wird zum einen verhindert, dass das Stechelement 7 an der Probenaufnahmezone 10 entlang reibt und dieses beschädigen kann, zum anderen, dass die Stechbewegung des Stechelementes 7 durch eine Reibung beeinflusst wird, insbesondere dass das Stechelement 7 abgebremst wird.

Figur 3b zeigt das Stechelement 7 von seiner Unterseite 22 betrachtet. Der Kapillarkanal 17 erstreckt sich von der Spitze 15 des Stechelements entlang des Nadelelements 14 bis zu seinem Grundkörper. Der Kapillarkanal 17 weist einen Probeneinlass 23 auf; durch den die Körperflüssigkeit nach dem Einstich in den Kapillarkanal 17 eindringen kann. Der Probeneinlass 23 ist an der Spitze 15 angeordnet. Am Ende des Kapillarkanals 17, das dem Pobeneinlass 23 gegenüber liegt, weist der Kapillarkanal 17 einen Probenauslass 24 auf, durch den die Körperflüssigkeit aus dem Kapillarkanal 17 austreten kann. In der gezeigten Ausführungsform ist der Kapillarkanal 17 einseitig offen, so dass ein rinnenartiger Kapillarkanal 17 gebildet wird. Die offene obere Seite des hier als Halbzylinder ausgebildeten Kapillarkanals 17 stellt somit den Probeneinlass 23 und den Probenauslass 24 dar, die ineinander übergehen. Der Probeneinlass 23 und der Probenauslass 24 sind also nicht nur auf die beiden Enden des Kapillarkanals 17 begrenzt.

Das in den Figuren 2 und 3 dargestellte Dispo zeichnet sich dadurch aus, dass es besonders flach ist. Es lässt sich gut stapeln und kann deshalb einfach magaziniert werden.

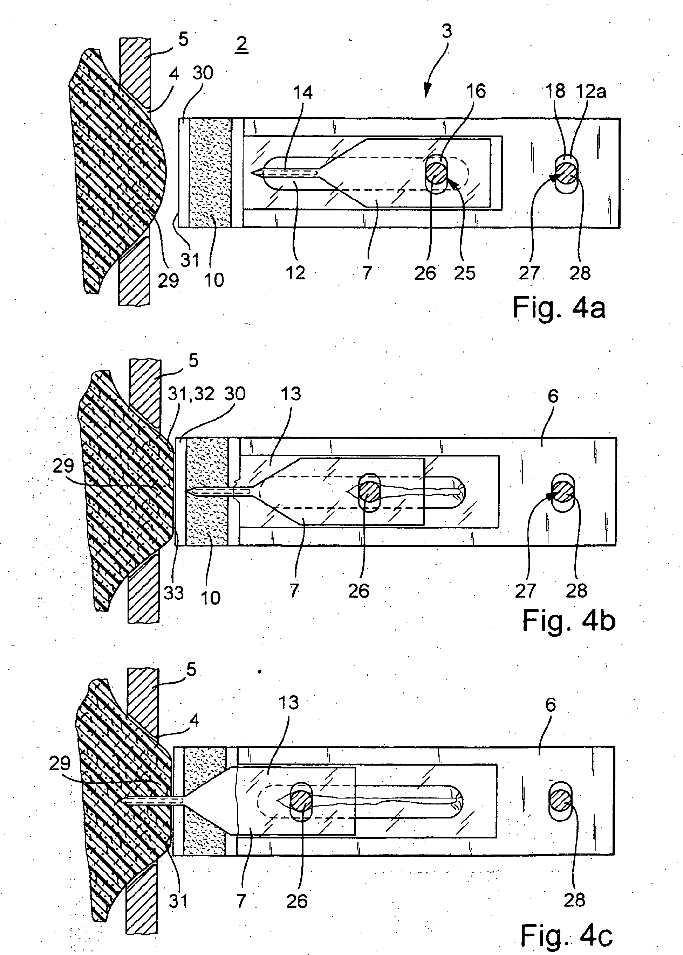

Die Figuren 4a bis h zeigen schematisch das vordere Ende des Analysegerätes 2 während unterschiedlicher Bewegungsphasen des Dispos 3.

Figur 4a zeigt das Dispo 3 in einer Kopplungsposition, nach dem das Dispo 3 aus einem (hier nicht dargestellten) Stapelmagazin entnommen worden ist. In der Kopplungsposition wird das Dispo 3 von einer Kopplungseinheit angekoppelt. Die Kopplungseinheit hat einen ersten Kopplungsmechanismus 25 zur Kopplung mit dem Stechelement 7, der in Form eines Dorns 26 ausgebildet ist. Der Dorn 26 greift durch die Kopplungsausnehmung 16 des Stechelements 7. Zugleich erstreckt sich der Dorn 26 in die Längsnut 12 des Teststreifens 6, in dem er sich während der Stechbewegung des Stechelements 7 bewegen kann. Beim Einführen des Dorns 26 in die Kopplungsausnehmung 16 und die Längsnut 12 wird die als Folie ausgebildete Schutzhülle 13 durchstochen. Bis zu diesem Zeitpunkt ist das Stechelement 7 steril in seiner Schutzhülle 13.

Ein zweiter Kopplungsmechanismus 27 ist bevorzugt ebenfalls als Dorn 28 ausgebildet. Der Dorn 28 wird, vorzugsweise zeitgleich mit dem Dorn 26, derart geführt, dass er in die Bohrung 18 der Schutzhülle 13 sowie die korrespondierende Bohrung 12a des Teststreifens einsticht. Hierdurch kann der Teststreifen 6 gehalten und geführt werden, so dass auch der Teststreifen 6 eine Bewegung in und entgegen der Einstichrichtung durchführen kann. Die Kopplungsmechanismen 25 und 27 der Kopplungseinheit ermöglichen das Ankoppeln des Dispos 3 an eine (hier nicht dargestellte) Antriebseinheit, mit der die Bewegung des Stechelements 7 und des Teststreifens 6 angetrieben wird.

Da die Schutzhülle 13 sehr dünn und wenig reissfest ist, bietet sie den Dornen 26,28 keinen Widerstand. Das Ankoppeln des Dispos 3 mit der Kopplungseinheit wird nicht behindert.

In der Kopplungsposition nach Figur 4a ist das Dispo 3 noch so weit von der Öffnung 4 des Gehäuses 5 entfernt, dass ein an der Öffnung 4 anliegendes Körperteil, beispielsweise eine Fingerkuppe 29, das sich in die Öffnung 4 einwölbt, nicht mit dem Dispo 3 in Berührung kommt. Selbstverständlich können das Gehäuse 5 des Analysesystems und die Öffnung 4 derart ausgebildet sein, dass sich die Fingerkuppe 29 nicht in den Innenraum des Analysegeräts 2 hineinwölbt.

Zu Beginn des Einstichvorgangs wird der Dorn 28 des zweiten Kopplungsmechanismus 27 in Richtung auf die Öffnung 4 zubewegt, wodurch der Teststreifen 6 so weit in Einstichrichtung verfahren wird, dass das vordere Ende 30 des Teststreifens 6 an der Fingerkuppe 29 anliegt. Dieses in Einstichrichtung vordere Ende 30 ist bevorzugt als Hautkontaktfläche 31 ausgebildet. In einer besonderen Ausführungsform ist die vordere Kante 32 des Teststreifens 6 die Hautkontaktfläche 31. Bevorzugt liegt die Hautkontaktfläche 31 wenigstens während eines Teils des Bewegungsweges des Stechelements an dem Körperteil an und dient der Vortriebsphase der Stechbewegung als Stechtefenreferenzelement. 33. Ein vorgegebener Wert der Stechtiefe wird durch den Abstand in Einstichrichtung zwischen der Hautkontaktfläche 31 und der Position der Spitze 15 des Stechelementes 7 am Umkehrpunkt der Stechbewegung bestimmt. Somit lässt sich eine gewünschte Einstichtiefe des Stechelements 7 in die Fingerkuppe 29 einstellen und verändern.

Nachdem oder gleichzeitig mit dem Bewegen des Dorns 28 wird der Dorn 26 des ersten Kopplungsmechanismus 25 ebenfalls in Einstichrichtung bewegt, dass das Stechelement 7 entlang eines Teils seines Bewegungswegs in Einstichrichtung bewegt wird. Die Schutzfolie 13 wird von dem Dorn 26 weiter aufgerissen. An ihrem vorderen Ende wird sie von der Spitze 15 des Stechelements 7 durchstochen. Bevorzugt wird das Stechelement 7 soweit in Einstichrichtung bewegt, bis die Spitze 15 des Stechelements 7 an der vorderen Kante der Probenaufnahmezone 10 liegt. Vorzugsweise ist die Spitze 15 etwa 0,5 mm von der vorderen Kante 32 des Teststreifens 6 entfernt so angeordnet, dass der Teststreifen 6 das Stechelement überragt.

In den Figuren 4a und 4b ist dargestellt, dass der Benutzer seine Fingerkuppe 29 an das Analysegerät 2 anlegt, bevor das Dispo 3 in seine Startposition (Figur 4b) bewegt wird. Alternativ kann auch zuerst die Bewegung des Dispos 3 von der Kopplungsposition in die Startposition erfolgen. Erst dann drückt der Benutzer seine Fingerkuppe 29 an die Öffnung 4 des Analysegerätes 2, bis die Fingerkuppe 29 an der Hautkontaktfläche 31 anliegt. Bevor ein Einstich erfolgen kann, muss beispielsweise elektronisch kontrolliert werden, dass die Fingerkuppe die Hautkontaktfläche 31 berührt. Optional kann in dieser Ausführungsform auch das Dispo 3 federnd in dem Analysegerät gelagert sein.

Während der in Figur 4c dargestellten Vortriebsphase des Einstichs wird der erste Kopplungsmechanismus 25 in der Längsnut 12 in Einstichrichtung bewegt, bis die Spitze 15 des Stechelements in die Fingerkuppe eindringt und eine Einstichwunde erzeugt. In dieser bevorzugten Ausführungsform wird also die Spitze 15 des Stechelements 7 während der Vortriebsphase der Stechbewegung über das in Einstichrichtung vordere. Ende 30 des Teststreifens 6 hinaus bewegt.

Sobald die eingestellte Stechtiefe erreicht ist, beginnt die Rückführungsphase des Stechelements 7, während der der Dorn 26 des Kopplungsmechanismus 25 entgegen der Einstichrichtung bewegt wird. Während des gesamten Einstichs, der mit einer sehr hohen Geschwindigkeit erfolgt, werden nur der Dorn 26 und das Stechelement 7 bewegt. Es findet also eine schnelle Relativbewegung des Stechelements 7 zum Teststreifen 6 statt, wobei der Dorn 26 relativ zu dem Dorn 28 bewegt wird. Bevorzugt wird während des Einstichs der Teststreifen 6 selbst nicht oder nur sehr langsam bewegt, so dass der Teststreifen 6 mit der Hautkontaktfläche 31 an der Fingerkuppe 29 anliegt und so einen Druck auf die Fingerkuppe ausübt.

Bevorzugt schließt sich an die Stechbewegung des Stechelements 7 eine Sammelbewegung an, während der die Relativbewegung zwischen dem Stechelement 7 und dem Teststreifen 6 im Vergleich zu der Stechbewegung deutlich langsamer ist. Alternativ findet gar keine Relativbewegung statt. Das Stechelement 7 und der Teststreifen 6 werden synchron miteinander bewegt. Alternativ kann der Teststreifen 6 des Dispo 3 in seiner Position verbleiben.

Wird während der Sammelbewegung der Teststreifen 6 ebenfalls in Einstichrichtung bewegt, so wird der von der Hautkontaktfläche 31 auf die Fingerkuppe ausgeübte Druck reduziert. Dadurch wird der Blutaustritt aus der Wunde gefördert. Das Blut kann in den Kapillarkanal 17 des Stechelements 7 eindringen. Auf diese Weise lässt sich ein effektives "Sammeln" von Blut realisieren. Ein manuelles und/oder mechanisches "Melken" ist nicht notwendig. Die Hautkontaktfläche 31 dient hierbei also als Expressionshilfe.

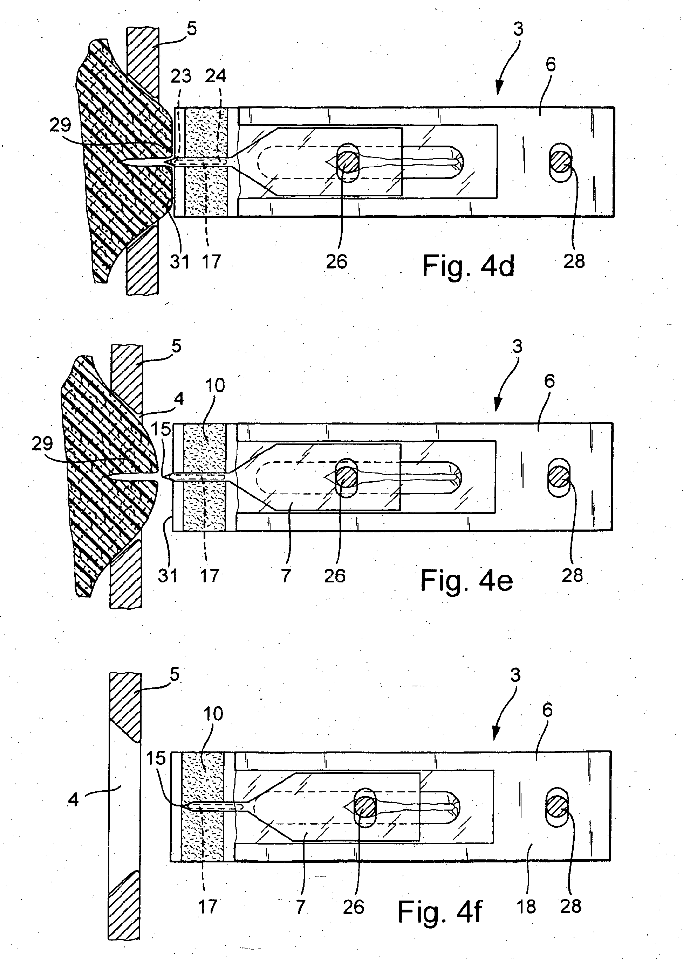

Während der in Figur 4d dargestellten Sammelbewegung des Stechelements tritt das Blut aus der Einstichwunde durch den Probeneinlass 23 in den Kapillarkanal 17 ein. Durch die Kapillarwirkung wird das Blut entlang des Kanals in Richtung auf den Probenauslass 24 zubewegt.

Am Ende der Sammelbewegung, die in Figur 4e dargestellt ist, ist das Dispo 3 so weit von der Öffnung 4 weg bewegt, dass kein Kontakt mehr zur Fingerkuppe 29 besteht. Die Fingerkuppe 29 kann von der Öffnung 4 weggenommen werden. Die Sammelbewegung ist abgeschlossen. Der Kapillarkanal 17 ist mit Blut gefüllt.

In dem gezeigten Beispiel weist die Spitze 15 des Stechelements 7 gegenüber dem Teststreifen 6 einen Überstand auf, der bevorzugt mindestens im letzten Teil der Sammelbewegung beibehalten wird.

Im Anschluss an die Sammelbewegung wird das Stechelement 7 in einer Transferbewegung in die Probenübertragungsposition bewegt, wie in Figur 4f dargestellt. Darin wird der Dorn 26 relativ zu dem Dorn 28 so weit bewegt, bis die Spitze 15 des Stechelements 7 in etwa mit dem vorderen Rand der Probenaufnahmezone 10 übereinstimmt.

Zusätzlich wird das Dispo 3 von der Gehäuseöffnung weg bewegt, bis es eine Position erreicht, in der die Probenaufnahmezone 10 im Bereich einer Andruckeinrichtung 34 des Analysegeräts 2 positioniert ist. Diese Bewegung wird durch eine Bewegung des Dorns 26 hervorgerufen. Die Bewegung des Dispos 3 kann vor, nach oder gleichzeitig mit der oben beschriebenen Bewegung des Stechelements 7 erfolgen.

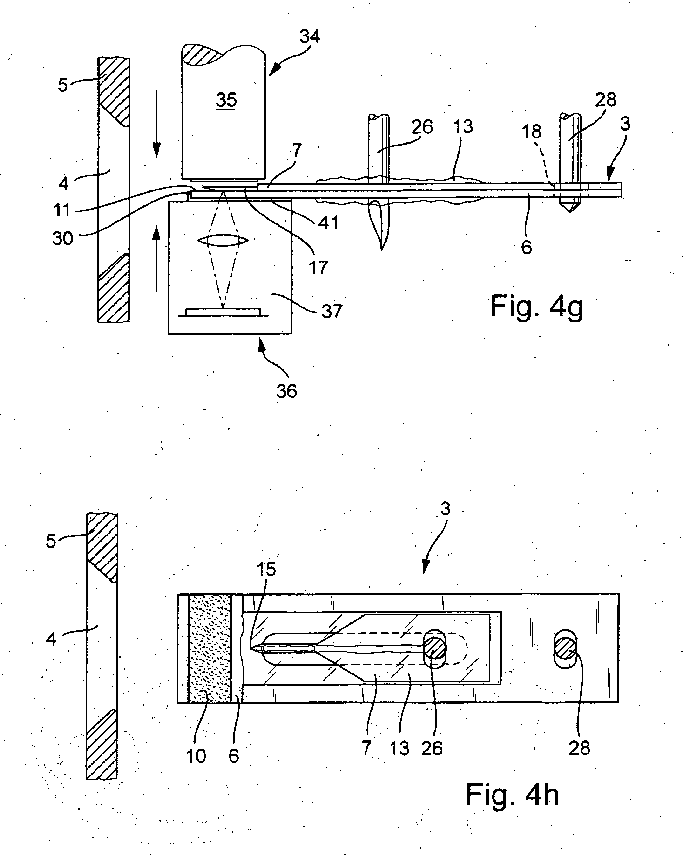

In der in den Figuren 4f und 4g dargestellten Probenübertragungsposition des Stechelements ist der Probenauslass 24 des Kapillarkanals 17 derart der Probenaufnahmezone 10 benachbart, dass das Blut aus dem Kapillarkanal 17 durch den Probenauslass 24 zu der Probenaufnahmezone 10 übertragen werden kann. Der Kapillarkanal 17 ist an der Unterseite des Stechelements 7 angeordnet, also an der der Probenkontaktseite 11 des Teststreifens 6 zugewandten Seite. Der Probenauslass 24 ist direkt oberhalb der Probenaufnahmezone 10 positioniert. Eine einfache Übertragung des Blutes ist möglich. Bevorzugt ist der Kapillarkanal 17 derart einseitig offen, dass seine Öffnung zu der Probenaufnahmezone 10 hin orientiert ist.

Bevorzugt ist das Stechelement 7 derart von dem Teststreifen 6 in Richtung der Senkrechten zur Probenkontaktseite 11 beabstandet, dass das Stechelemenf 7 die Probenaufnahmezone 10 nicht berührt. Das Stechelement 7 wird nun in der Probenübertragungsposition von oben (in Pfeilrichtung) durch einen Stempel 35 der Andruckeinrichtung 34 an die Probenaufnahmezone 10 angedrückt. Der Probenauslass 24, der von einem Teil der einseitigen Öffnung des Kapillarkanals 17 gebildet wird, wird an die Probenaufnahmezone 10 so bewegt, dass das Blut zuverlässig übertragen wird.

Der Andruckeinrichtung 34 gegenüber (unterhalb des Dispos 3) ist eine Mess- und Auswerteeinheit 36 angeordnet, die bevorzugt eine optische Messeinrichtung 37 umfasst. Die optische Messeinrichtung 37 ist vorteilhafterweise an der Unterseite des Teststreifens 6 angeordnet. Sie dient zur Durchführung einer photometrischen Messung, um eine optisch messbare Messgröße zu messen, die für die Bestimmung des Analyten in der Körperflüssigkeit bzw. in dem Blut charakteristisch ist.

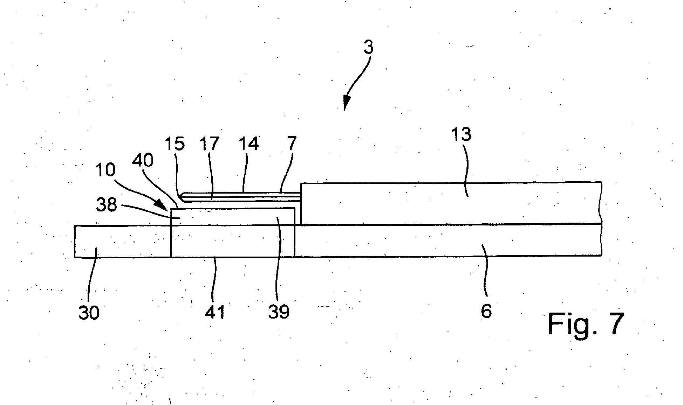

Figur 7 zeigt im Detail das vordere Ende 30 des Dispos 3 mit der Probenaufnahmezone 10, an der die Messeinrichtung 37 anliegt. Die Probenaufnahmezone 10 schließt vorzugsweise ein Testfeld 38 ein, das mindestens eine saugfähige Schicht 39 umfasst. Das Testfeld 38 hat eine Probenaufnahmefläche 40 auf der Probenkontaktseite 11 des Teststreifens 6 und eine Detektionsfläche 41 auf der Unterseite, die der Probenkontaktseite 11 gegenüber liegt. In der bevorzugten Ausführungsform des Dispos 3 sind die Probenaufnahmezone 10 und das Testfeld 36 identisch. Es ist jedoch auch möglich, dass das Testfeld 38 und die Probenaufnahmezone 10 voneinander abweichen, so dass die Detektionsfläche 41 zur optischen Messung der charakteristischen Messgröße räumlich von der Probenaufnahmefläche 40 getrennt ist.

Vorzugsweise findet die photometrische Messung mittels der optischen Messeinrichtung 37 durch diffuse Reflexion an der Detektionsfläche 41 statt. Die Detektionsfläche 41 steht besonders bevorzugt in Fluidverbindung mit dem Testfeld 38 bzw. der Probenaufnahmezone 10. Unter diffuser Reflexion wird eine Messung mit einer Messanordnung verstanden, bei der der Einfallswinkel einer (elektromagnetischen) Welle (z. B. Licht) von dem Austrittswinkel nach Auftreffen auf die Detektionsfläche 41 verschieden ist.

Da die optische Messung an der Unterseite, also an der der Probenkontaktseite 11 des Teststreifens 6 gegenüberliegenden Seite stattfindet, verfälschen Farbstoffe, die beispielsweise im Blut enthalten sind, das Messergebnis nicht. Diese Farbstoffe verbleiben in der saugfähigen Schicht 39 bzw. an der Probenaufnahmefläche 40. Geeignete Ausführungen derartiger Testfelder 38 sind im Stand der Technik allgemein bekannt.

Nachdem in der Probenübertragungsposition des Stechelements 7 die photometrische Messung durchgeführt wurde, wird das Stechelement 7 relativ zu dem Teststreifen 6 soweit in Richtung hinteres Ende des Dispos 3 bewegt, bis das Stechelement 7 wieder in seiner Ausgangslage angeordnet ist, in der es vollständig in die (teilweise beschädigte) Schutzhülle 13 zurückgezogen ist, Figur 4h.

Das Dispo 3 wird dann in die Kopplungsposition zurückbewegt, aus der es im weiteren durch eine Transporteinrichtung in das Stapelmagazin zurück- bzw in einen Aufnahmebehälter für verbrauchte Dispos bewegt werden kann.

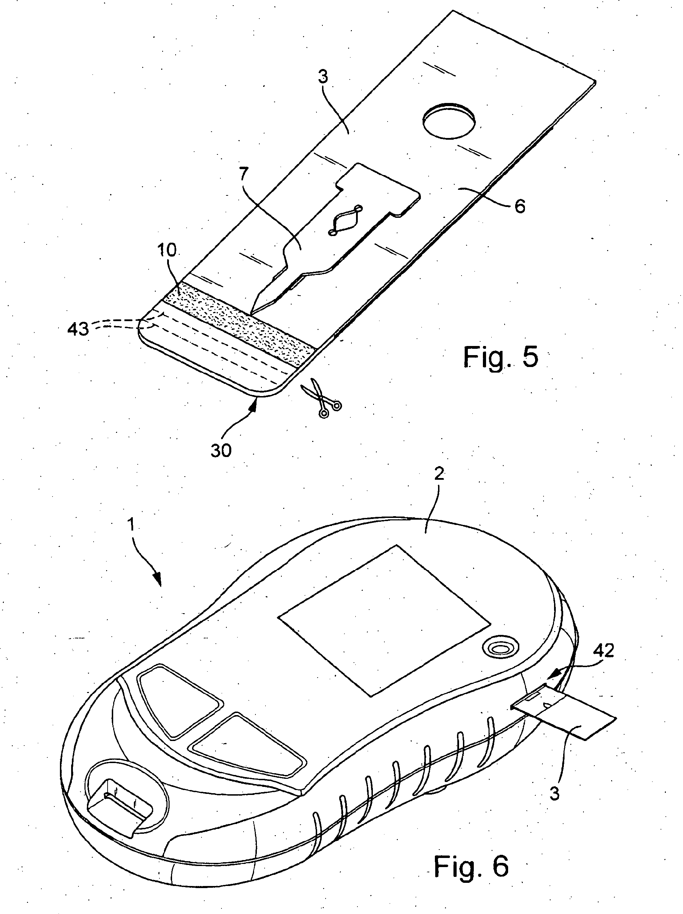

Figur 5 zeigt eine schematische Darstellung einer weiteren Ausführungsform eines Dispos 3. Der Teststreifen 6 des Dispos ist vorzugsweise so ausgebildet, dass die Länge des Dispos, also die Abmessung in Einstichrichtung, durch Abknicken oder Abschneiden des vorderen Endes 30 des Teststreifens 6 variiert werden kann. Damit lassen sich unterschiedliche Stechtiefen einstellen, ohne dass eine weitere Stechtiefeneinstellvorrichtung im Analysegerät 2 vorzusehen ist. Am vorderen Ende 30 bzw. in dem Bereich des vorderen Endes 30 sind mehrere Markierungen und/oder Perforierungen des Teststreifens 6 angeordnet, an denen das Dispo 3 derart gekürzt werden kann, dass die Stechtiefe beim Einstichvorgang individuell eingestellt werden kann. Jede Markierung bzw. Perforierung entspricht dabei einer vorbestimmten Stechtiefe. Somit erfolgt die individuelle Stechtiefeneinstellung durch eine Längenänderung am Dispo 3.

In einer bevorzugten Ausführungsform ist auch bei dieser Art Dispo eine Probenaufnahmezone 10 vorgesehen, wobei die Markierungen bzw. Perforationen zum Kürzen des Dispos nicht bis in die Probenaufnahmezone 10 heranreichen. Selbst bei Einstellen der größten Stechtiefe (kürzester Teststreifen 6) ist die Probenaufnahmezone 10 von der vorderen Kante 33 des Dispos beabstandet.

Um eine möglichst genaue Einstellung der Stechtiefe zu erzielen, ist in einer Ausführungsform des Analysesystems 1 nach Figur 6 eine Kürzungseinheit 42 vorgesehen, die beispielsweise seitlich in dem Analysesystem 1 integriert ist. Die bevorzugte Kürzungseinheit 42 dient dazu, den Teststreifen 6 an seinem in Einstichrichtung vorderen Ende 30 zu kürzen, um die gewünschte Stechtiefe beim Einstich einzustellen.

Alternativ lässt sich die Kürzungseinheit 42 derart in das Analysesystems integrieren, dass ein aus einem Stapelmagazin transportiertes Dispo 3 automatisch auf eine einstellbare Länge gekürzt werden kann. Die gewünschte Länge, die mit einer Stechtiefe korrespondiert, kann z.B. vom Benutzer des Analysegerätes an einer Einstellvorrichtung eingestellt werden.

## Patentansprüche

1. Disposibles integriertes Probengewinnungs- und Analyseelement, welches ein Stechelement (7) mit einer Spitze (15) zum Erzeugen einer Einstichwunde in einem Körperteil und einen flachen Teststreifen (6) mit einer Probenaufnahmezone (10) einschließt, in die eine Körperflüssigkeitsprobe zur Durchführung einer Analyse übertragen wird, wobei
- die Probenaufnahmezone (10) des Teststreifens (6) an einer seiner Flachseiten (9), die eine Probenkontaktseite (11) bildet, angeordnet ist
- das Stechelement (7) parallel zu dem Teststreifen (6) angeordnet und relativ zu dem Teststreifen (6) auf einen Bewegungsweg bewegbar ist,
- das Stechelement (7) benachbart zu der Probenkontaktseite (11) des Teststreifens (6) angeordnet ist,
- das Stechelement (7) einen Kapillarkanal (17) aufweist, der einen Probeneinlass (23), durch den die Körperflüssigkeit nach dem Einstich in den Kapillarkanal (17) eintreten kann, und einen Probenauslass (24), durch den die Körperflüssigkeit aus dem Kapillarkanal (17) austreten kann, hat,
- das Stechelement (7) in eine Probenübertragungsposition bewegbar ist, in der der Probenauslass (24) derart mit der Probenaufnahmezone (10) des Teststreifens (6) benachbart ist, dass die Körperflüssigkeit zu der Probenaufnahmezone (10) übertragen werden kann, und
- der Teststreifen (6) etwa mittig eine Längsnut (12) aufweist, die sich in Längsrichtung erstreckt und als Durchgriff eines ersten Kopplungsmechanismus (25) dient, der das Stechelement (7) bewegt,
**dadurch gekennzeichnet, dass**
- die Probenaufnahmezonen (10) zu dem in Einstichrichtung vorderen Ende (30) des Teststreifens benachbart ist,
- das Stechelement (7) in Einstichrichtung derart hinter der Probenaufnahmezone (10) positioniert ist, dass die Probenaufnahmezone (10) zwischen dem Stechelement (7) und dem vorderen Ende (30) des Teststreifens (6) angeordnet ist, und
- der Teststreifen (6) im Bereich seines fernen Endes eine Bohrung (12a) zur Ankopplung eines zweiten Kopplungsmechanismus (27) an den Teststreifen (6) aufweist, um den Testreifen (6) in einem Analysegerät (2) zu bewegen.

2. Disposibles integriertes Probengewinnungs- und Analyseelement nach Anspruch 1, **dadurch gekennzeichnet, dass** der Kapillarkanal (17) des Stechelements (7) eine in radialer Richtung offene, sich mindestens über einen Teil seiner Länge erstreckende Längsöffnung aufweist, die bevorzugt zur Probenkontaktseite (11) des Teststreifens (6) hin orientiert ist.

3. Disposibles integriertes Probengewinnungs- und Analyseelement nach Anspruch 1 oder 2, **dadurch gekennzeichnet, dass** das Stechelement (7) von einer bei der Stechbewegung durchstechbaren Schutzhülle (13) umschlossen ist.

4. Disposibles integriertes Probengewinnungs- und Analyseelement nach einem der Ansprüche 1 bis 3, **dadurch gekennzeichnet, dass** das Stechelement (7) mindestens auf seinen Längsseiten von Abstandshaltern (21) umgeben ist, deren Dicke mindestens so groß ist, wie die Dicke des Stechelements (7).

5. Disposibles integriertes Probengewinnungs- und Analyseelement nach einem der Ansprüche 1 bis 4, **dadurch gekennzeichnet, dass** das Stechelement (7) flach ausgebildet ist und eine der Probenkontaktseite (11) zugewandte Unterseite (22) des Stechelements (7) und die Probenaufnahmezone (10) derart beabstandet sind, dass das Stechelement (7) wenigstens während der Vortriebsphase der Stechbewegung die Probenaufnahmezone (10) nicht berührt.

6. Disposibles integriertes Probengewinnungs- und Analyseelement nach einem der Ansprüche 1 bis 5, **dadurch gekennzeichnet, dass** der Teststreifen (6) dazu ausgebildet ist, dass seine Länge durch Abknicken oder Abschneiden des vorderen Endes (30) des Teststreifens (6) zu variiert wird, um unterschiedliche Stechtiefen einzustellen.

7. Analysesystem zur Bestimmung eines Analyten in einer Körperflüssigkeit, umfassend ein disposibles integriertes Probengewinnungs- und Analyseelement nach einem der vorhergehenden Ansprüche und ein wiederverwendbares Analysegerät (2).
wobei
- das disposible integrierte Probengewinnungs- und Analyseelement ein Stechelement (7) mit einer Spitze (15) zum Erzeugen einer Einstichwunde in einem Körperteil und einen flachen Teststreifen (6) mit einer Probenaufnahmezone (10) einschließt, in die eine Körperflüssigkeitsprobe zur Durchführung einer Analyse übertragen wird, und
- das wiederverwendbare Analysegerät (2) eine Kopplungseinheit einschließt, die dazu ausgebildet ist, ein integriertes Probengewinnungs- und Analyseelement mit einem Antrieb zu koppeln, durch den eine Stechbewegung des Stechelementes (7) angetrieben wird, bei dem es auf einen Einstichweg während einer. Vortriebsphase in Einstichrichtung und nach Erreichen eines Umkehrpunktes der Stechbewegung während einer Rückführungsphase entgegen der Einstichrichtung bewegt wird, und das eine Mess- und Auswerteeinheit (36) zur Messung einer für die Bestimmung des Analyten charakteristischen Messgröße und Ermittlung eines gewünschten Analyseergebnisses auf der Grundlage der Messung einschließt,
wobei
- die Probenaufnahmezone (10) des Teststreifens (6) an einer seiner Flachselten (9), die eine Probenkontaktseite (11) bildet, angeordnet ist, und
- das Stechelement (7) parallel zu dem Teststreifen (6) angeordnet und während mindestens eines Teils der Einstichbewegung relativ zu dem Teststreifen (6) auf einem Bewegungsweg bewegbar ist,
- das Stechelement (7) benachbart zu der Probenkontaktseite (11) des Teststreifens (6) angeordnet ist,
- das Stechelement (7) einen Kapillarkanal (17) aufweist, der einen Probeneinlass (23), durch den die Körperflüssigkeit nach dem Einstich in den Kapillarkanal (17) eintreten kann, und einen Probenauslass (24), durch den die Körperflüssigkeit aus dem Kapillarkanal (17) austreten kann, hat, und
- der Bewegungsweg des Stechelements (7) eine Probenübertragungsposition einschließt, in der der Probenauslass (24) derart der Probenaufnahmezone (10) des Teststreifens (6) benachbart ist, dass die Körperflüssigkeit aus dem Kapillarkanal (17) durch den Probenauslass (24) zu der Probenaufnahmezone (10) übertragen werden kann
**dadurch gekennzeichnet, dass**
- die Probenaufnahmezonen (10) zu dem in Einstichrichtung vorderen Ende (30) des Teststreifens benachbart ist, und
- das Stechelement (7) in Einstichrichtung derart hinter der Probenaufnahmezone (10) positioniert ist, dass die Probenaufnahmezone (10) zwischen dem Stechelement (7) und dem vorderen Ende (30) des Teststreifens (6) angeordnet ist, und
- dass die Kopplungseinheit einen ersten Kopplungsmechanismus (25) zur Kopplung mit dem Stechelement (7) und zweiten Kopplungsmechanismus (27) zur Kopplung mit dem Teststreifen (6) umfasst.

8. Analysesystem nach Anspruch 7 oder disposibles integriertes Probengewinnungs- und Analyseelement nach einem der Ansprüche 1 bis 6, **dadurch gekennzeichnet, dass** die Mess- und Auswerteeinheit (36) eine optische Messeinrichtung (37) umfasst, um mittels einer photometrischen Messung eine optisch messbare, für die Bestimmung des Analyten charakteristische Messgröße zu messen.

9. Analysesystem nach Anspruch 7 oder 8 oder disposibles integriertes Probengewinnungs- und Analyseelement nach einem der Ansprüche 1 bis 6, **dadurch gekennzeichnet, dass** die Probenaufnahmezone (10) ein Testfeld (38) enthält dass mindestens eine saugfähige Schicht umfasst und eine Probenaufnahmefläche (40) auf der Probenkontaktseite (11) des Teststreifens (6) und eine Detektionsfläche (41) auf der der Probenkontaktseite (11) gegenüberliegenden Seite des Teststreifens (6) hat.

10. Analysesystem nach einem der Ansprüche 7 bis 9 oder disposibles integriertes Probengewinnungs- und Analyseelement nach einem der Ansprüche 1 bis 6, **dadurch gekennzeichnet, dass** die Spitze (15) des Stechelements (7) während der Vortriebsphase der Stechbewegung über das in Einstichrichtung vordere Ende (30) des Teststreifens (6) hinausbewegt wird.

11. Analysesystem nach einem der Ansprüche 7 bis 10, **dadurch gekennzeichnet, dass** sich an die Stechbewegung des Stechelementes (7) eine Sammelbewegung anschließt, in der eine im Vergleich zu der Stechbewegung langsamere oder keine Relativbewegung des Stechelements (7) relativ zu dem Teststreifen (6) stattfindet.

12. Analysesystem nach einem der Ansprüche 10 und 11, **dadurch gekennzeichnet, dass** während der Sammelbewegung eine Körperflüssigkeitsprobe in den Kapillarkanal (17) des Stechelements (7) aufgenommen wird.

13. Analysesystem nach einem der Ansprüche 7 bis 12, **dadurch gekennzeichnet, dass** das Analysegerät (2) eine Andruckeinrichtung (34) aufweist, mittels der das Stechelement (7) in der Probenübertragungsposition an die Probenaufnahmezone (10) angedrückt wird, um die Körperflüssigkeit zu der Probenaufnahmezone (10) zu übertragen.

14. Analysesystem nach einem der Ansprüche 7 bis 13, **dadurch gekennzeichnet, dass** das in Einstichrichtung vordere Ende (30) des Teststreifens (6) eine Hautkontaktfläche (31) aufweist und als Stechtiefenreferenzelement (33) dient, wobei ein vorgegebener Wert der Stechtiefe durch den Abstand in Einstichrichtung zwischen der Hautkontaktfläche (31) und der Position der Spitze (15) des Stechelementes (7) an dem Umkehrpunkt der Stechbewegung bestimmt wird.

15. Analysesystem nach Anspruch 14, **dadurch gekennzeichnet, dass** in dem Analysesystem (1) eine Kürzungseinheit (42) integriert ist, um den Teststreifen (6) an seinem in Einstichrichtung vorderen Ende (30) zu kürzen.

16. Analysesystem nach einem der Ansprüche 7 bis 15, **dadurch gekennzeichnet, dass** die Kopplungseinheit einen ersten Kopplungsmechanismus (25) zur Kopplung mit dem Stechelement (7) und zweiten Kopplungsmechanismus (27) zur Kopplung mit dem Teststreifen (6) umfasst.

17. Analysesystem nach einem der Ansprüche 7 bis 16, **dadurch gekennzeichnet, dass** mehrere Probengewinnungs- und Analyseelemente in einem Stapelmagazin gelagert sind, die mittels einer Transporteinrichtung einzeln dem Stapelmagazin entnommen und in eine Kopplungsposition bewegt werden können.

## Claims

1. Disposable integrated sample acquisition and analysis element comprising a piercing element (7) with a tip (15) for generating a bodily puncture wound, and a flat test strip (6) having a sample receiving zone (10) to receive a body fluid sample for analysis,
wherein
- the sample receiving zone (10) of the test strip (6) is located on one of its flat sides (9), which forms a sample contact side (11),
- the piercing element (7) is located parallel to the test strip (6) and is movable relative to the test strip (6) on a movement path,
- the piercing element (7) is adjacent to the sample contact side (11) of the test strip (6),
- the piercing element (7) has a capillary channel (17) comprising, a sample inlet (23) for body fluid entry after the piercing into the capillary channel (17), and a sample outlet (24) for body fluid exit from the capillary channel (17),
- the piercing element (7) is movable into a sample transfer position, wherein the sample outlet (24) is adjacent to the sample receiving zone (10) of the test strip (6) such that body fluid may be transferred to the sample receiving zone (10), and
- the test strip (6) has a longitudinal groove (12) positioned approximately at the centre of the test strip, the groove (12) extending in a longitudinal direction and acting as a passage of a first coupling mechanism (25), which moves the piercing element (7),
**characterised in that**
- the sample receiving zone (10) is adjacent to the front end (30) of the test strip in a puncture direction,
- the piercing element (7) is positioned in the puncture direction behind the sample receiving zone (10) in such a manner that the sample receiving zone (10) is located between the piercing element (7) and the front end (30) of the test strip (6), and
- at its remote end, the test strip (6) has a bore (12a) for coupling a second coupling mechanism (27) to the test strip (6) in order to move the test strip (6) in an analysis instrument (2).

2. Disposable integrated sample acquisition and analysis element according to claim 1, **characterised in that** the capillary channel (17) of the piercing element (7) has a longitudinal opening that opens in a radial direction that extends over at least a part of its length, wherein the opening is preferably oriented towards the sample contact side (11) of the test strip (6).

3. Disposable integrated sample acquisition and analysis element according to claims 1 or 2, **characterised in that** the piercing element (7) is enclosed with a protective cover (13), which can be punctured during the piercing movement.

4. Disposable integrated sample acquisition and analysis element according to one of the claims 1 to 3, **characterised in that** the piercing element (7) is enclosed at least on its longitudinal sides by spacers (21), wherein the spacers have a thickness that is at least as great as the thickness of the piercing element (7).

5. Disposable integrated sample acquisition and analysis element according to one of the claims 1 to 4, **characterised in that** the piercing element (7) is flat, and a bottom side (22) of the piercing element (7) facing towards the sample contact side (11) and the sample receiving zone (10) are spaced apart in such a manner that the piercing element (7) does not touch the sample receiving zone (10) at least during the propulsion phase of the piercing movement.

6. Disposable integrated sample acquisition and analysis element according to one of the claims 1 to 5, **characterised in that** the test strip (6) is designed so that its length can be varied by breaking or cutting off the front end (30) of the test strip (6) to set different piercing depths.

7. Analysis system for determining an analyte in a body fluid, comprising a disposable integrated sample acquisition and analysis element according to any one of the preceding claims, and a reusable analysis instrument (2),
wherein
- the disposable integrated sample acquisition and analysis element comprising a piercing element (7) with a tip (15) for generating a bodily puncture wound, and a flat test strip (6) having a sample receiving zone (10) to receive a body fluid sample for analysis, and
- the reusable analysis instrument (2) comprises a coupling unit adapted to couple an integrated sample acquisition and analysis element to a drive for driving the piercing element (7) in a piercing movement, wherein during a propulsion phase the piercing element is moved on a puncture path in the puncture direction, and during a retraction phase the piercing element is moved opposite to the puncture direction after reaching a reversal point of the piercing movement; and the analysis instrument (2) comprises a measuring and analysis unit (36) for measuring a measurement variable characteristic for the determination of the analyte and for determining a desired analysis result on the basis of the measurement,
wherein
- the sample receiving zone (10) of the test strip (6) is located on one of its flat sides (9), which forms a sample contact side (11), and
- the piercing element (7) is located parallel to the test strip (6) and is movable relative to the test strip (6) on a movement path during at least a part of the puncture movement,
- the piercing element (7) is located adjacent to the sample contact side (11) of the test strip (6),
- the piercing element (7) comprises a capillary channel (17), which has a sample inlet (23), through which the body fluid may enter after the piercing into the capillary channel (17), and a sample outlet (24),
through which the body fluid may exit from the capillary channel (17), and
- the movement path of the piercing element (7) comprises a sample transfer position, in which the sample outlet (24) is adjacent to the sample receiving zone (10) of the test strip (6) in such a manner that the body fluid may be transferred from the capillary channel (17) through the sample outlet (24) to the sample receiving zone (10), **characterized in that**
- the sample receiving zone (10) is adjacent to the front end (30) of the test strip in the puncture direction, and
- the piercing element (7) is positioned in the puncture direction behind the sample receiving zone (10) in such a manner that the sample receiving zone (10) is located between the piercing element (7) and the front end (30) of the test strip (6), and
- that the coupling unit comprises a first coupling mechanism (25) for coupling to the piercing element (7) and a second coupling mechanism (27) for coupling to the test strip (6).

8. Analysis system according to claim 7, or disposable integrated sample acquisition and analysis element according to any one of the claims 1 to 6, **characterized in that** the measuring and analysis unit (36) comprises an optical measuring apparatus (37), for measuring by means of a photometric measurement an optically measurable measurement variable which is characteristic for the determination of the analyte.

9. Analysis system according to claim 7 or 8, or disposable integrated sample acquisition and analysis element according to any one of the claims 1 to 6, **characterized in that** the sample receiving zone (10) contains a test field (38), comprising at least one absorbing layer and having a sample receiving surface (40) on the sample contact side (11) of the test strip (6) and a detection surface (41) on the side of the test strip (6) opposite to the sample contact side (11).

10. Analysis system according to any one of the claims 7 to 9, or disposable integrated sample acquisition and analysis element according to any one of the claims 1 to 6, **characterized in that** the tip (15) of the piercing element (7) is moved during the propulsion phase of the piercing movement beyond the front end (30) of the test strip (6) in the puncture direction.

11. Analysis system according to any one of the claims 7 to 10, **characterized in that** a collection movement follows the piercing movement of the piercing element (7), in which a relative movement slower than the piercing movement or no relative movement of the piercing element (7) relative to the test strip (6) takes place.

12. Analysis system according to any one of the claims 10 and 11, **characterized in that**, during the collection movement, a body fluid sample is received in the capillary channel (17) of the piercing element (7).

13. Analysis system according to any one of the claims 7 to 12, **characterized in that** the analysis instrument (2) has a contact pressure device (34), by which the piercing element (7) is pressed against the sample receiving zone (10), when in the sample transfer position, thereby to transfer the body fluid to the sample receiving zone (10).

14. Analysis system according to any one of the claims 7 to 13, **characterized in that** the front end (30) of the test strip (6) in the puncture direction comprises a skin contact surface (31) and is used as a piercing depth reference element (33), a predetermined value of the piercing depth being determined by the spacing in the puncture direction between the skin contact surface (31) and the position of the tip (15) of the piercing element (7) at the reversal point of the piercing movement.

15. Analysis system according to claim 14, **characterized in that** a shortening unit (42) is integrated in the analysis system (1), for reducing the length of the test strip (6) at its front end (30) in the puncture direction.

16. Analysis system according to any one of the claims 7 to 15, **characterized in that** the coupling unit comprises a first coupling mechanism (25) for coupling to the piercing element (7) and a second coupling mechanism (27) for coupling to the test strip (6).

17. Analysis system according to any one of the claims 7 to 16, **characterized in that** a plurality of sample acquisition and analysis elements are stored in a stacking magazine, which elements may be removed individually from the stacking magazine and moved into a coupling position by means of a transport apparatus.

## Revendications

1. Elément de prélèvement d'échantillon et d'analyse intégré jetable, comprenant un élément piquant (7) doté d'une pointe (15) servant à pratiquer une incision dans une partie du corps, et une bandelette de test (6) plate dotée d'une zone de réception d'échantillon (10) dans laquelle un échantillon de liquide corporel est transféré en vue d'effectuer une analyse,
dans lequel
- la zone de réception d'échantillon (10) de la bandelette de test (6) est agencée sur l'un de ses côtés plats (9) qui forme un côté (11) en contact avec l'échantillon,
- l'élément piquant (7) est disposé parallèlement à la bandelette de test (6) et peut être déplacé par rapport à la bandelette de test (6) sur un trajet de déplacement,
- l'élément piquant (7) est positionné de manière adjacente au côté (11) en contact avec l'échantillon de la bandelette de test (6),
- l'élément piquant (7) présente un canal capillaire (17) qui possède une entrée d'échantillon (23) à travers laquelle le liquide corporel peut entrer dans le canal capillaire (17) après la piqûre, et une sortie d'échantillon (24) à travers laquelle le liquide corporel peut sortir du canal capillaire (17),
- l'élément piquant (7) peut être amené dans une position de transfert d'échantillon dans laquelle la sortie d'échantillon (24) est adjacente à la zone de réception d'échantillon (10) de la bandelette de test (6), de sorte que le liquide corporel peut être transféré vers ladite zone de réception d'échantillon (10),
- la bandelette de test (6) présente sensiblement au milieu une rainure longitudinale (12) qui s'étend dans le sens de la longueur et qui sert de passage pour un premier mécanisme de couplage (25) qui assure le déplacement de l'élément piquant (7),
**caractérisé en ce que**
- la zone de réception d'échantillon (10) est adjacente à l'extrémité avant (30) de la bandelette de test dans le sens de la piqûre,
- l'élément piquant (7) est positionné dans le sens de la piqûre derrière la zone de réception d'échantillon (10), de sorte que ladite zone de réception d'échantillon (10) est placée entre l'élément piquant (7) et l'extrémité avant (30) de la bandelette de test (6), et
- la bandelette de test (6) présente au niveau de son extrémité distale un perçage (12a) permettant de coupler un second mécanisme de couplage (27) à la bandelette de test (6) afin de déplacer ladite bandelette de test (6) dans un appareil d'analyse (2).

2. Elément de prélèvement d'échantillon et d'analyse intégré jetable selon la revendication 1, **caractérisé en ce que** le canal capillaire (17) de l'élément piquant (7) présente un orifice longitudinal ouvert dans le sens radial et s'étendant au moins sur une partie de sa longueur, lequel orifice est de préférence orienté vers le côté (11) en contact avec l'échantillon de la bandelette de test (6).

3. Elément de prélèvement d'échantillon et d'analyse intégré jetable selon la revendication 1 ou 2, **caractérisé en ce que** l'élément piquant (7) est entouré d'une enveloppe protectrice (13) pouvant être percée lors du mouvement de piqûre.

4. Elément de prélèvement d'échantillon et d'analyse intégré jetable selon l'une des revendications 1 à 3, **caractérisé en ce que** l'élément piquant (7) est entouré, au moins sur ses côtés longitudinaux, d'entretoises (21) dont l'épaisseur est au moins égale à l'épaisseur de l'élément piquant (7).

5. Elément de prélèvement d'échantillon et d'analyse intégré jetable selon l'une des revendications 1 à 4, **caractérisé en ce que** l'élément piquant (7) est de conformation plate, et un côté inférieur (22) tourné vers le côté (11) en contact avec l'échantillon de l'élément piquant (7) et la zone de réception d'échantillon (10) sont espacés de sorte que l'élément piquant (7), au moins pendant la phase de propulsion du mouvement de piqûre, ne touche pas la zone de réception d'échantillon (10).

6. Elément de prélèvement d'échantillon et d'analyse intégré jetable selon l'une des revendications 1 à 5, **caractérisé en ce que** la bandelette de test (6) est adaptée pour faire varier sa longueur, par pliage ou coupe de l'extrémité avant (30) de la bandelette de test (6), en vue de régler différentes profondeurs de piqûre.

7. Système d'analyse pour la détermination d'un analyte dans un liquide corporel, comprenant un élément de prélèvement d'échantillon et d'analyse intégré jetable selon l'une des revendications précédentes et un appareil d'analyse réutilisable (2),
dans lequel
- l'élément de prélèvement d'échantillon et d'analyse intégré jetable comprend un élément piquant (7) doté d'une pointe (15) servant à pratiquer une incision dans une partie du corps, et une bandelette de test (6) plate dotée d'une zone de réception d'échantillon (10) dans laquelle un échantillon de liquide corporel est transféré en vue d'effectuer une analyse, et
- l'appareil d'analyse réutilisable (2) comprend une unité de couplage qui est adaptée pour coupler un élément de prélèvement d'échantillon et d'analyse intégré jetable avec un mécanisme d'entraînement permettant de commander un mouvement de piqûre de l'élément piquant (7), opération au cours de laquelle ledit élément est déplacé, pendant une phase de propulsion, sur un trajet de piqûre dans le sens de la piqûre et, après avoir atteint un point de retour du mouvement de piqûre, est déplacé, pendant une phase de retour, en sens opposé au sens de la piqûre, et comprend une unité de mesure et d'évaluation (36) servant à mesurer une grandeur de mesure caractéristique pour la détermination de l'analyte et à déterminer un résultat d'analyse souhaité sur la base de cette mesure,
dans lequel
- la zone de réception d'échantillon (10) de la bandelette de test (6) est agencée sur l'un de ses côtés plats (9) qui forme un côté (11) en contact avec l'échantillon, et
- l'élément piquant (7) est disposé parallèlement à la bandelette de test (6) et peut être déplacé par rapport à la bandelette de test (6) sur un trajet de déplacement au moins pendant une partie du mouvement de piqûre,
- l'élément piquant (7) est positionné de façon adjacente au côté (11) en contact avec l'échantillon de la bandelette de test (6),
- l'élément piquant (7) présente un canal capillaire (17) doté d'une entrée d'échantillon (23) à travers laquelle le liquide corporel peut entrer dans le canal capillaire (17) après la piqûre, et d'une sortie d'échantillon (24) à travers laquelle le liquide corporel peut sortir du canal capillaire (17), et
- le trajet de déplacement de l'élément piquant (7) comprend une position de transfert d'échantillon dans laquelle la sortie d'échantillon (24) est adjacente à la zone de réception d'échantillon (10) de la bandelette de test (6) de sorte que le liquide corporel peut être transféré du canal capillaire (17), à travers la sortie d'échantillon (24), vers ladite zone de réception d'échantillon (10),
**caractérisé en ce que**
- la zone de réception d'échantillon (10) est adjacente à l'extrémité avant (30) de la bandelette de test dans le sens de la piqûre, et
- l'élément piquant (7) est positionné dans le sens de la piqûre derrière la zone de réception d'échantillon (10) de sorte que ladite zone de réception d'échantillon (10) est placée entre l'élément piquant (7) et l'extrémité avant (30) de la bandelette de test (6), et
- **en ce que** l'unité de couplage comprend un premier mécanisme de couplage (25) pour le couplage avec l'élément piquant (7) et un second mécanisme de couplage (27) pour le couplage avec la bandelette de test (6).

8. Système d'analyse selon la revendication 7 ou élément de prélèvement d'échantillon et d'analyse intégré jetable selon l'une des revendications 1 à 6, **caractérisé en ce que** l'unité de mesure et d'évaluation (36) comprend un dispositif de mesure optique (37) afin de mesurer, au moyen d'une mesure photométrique, une grandeur de mesure optiquement mesurable et caractéristique pour la détermination de l'analyte.

9. Système d'analyse selon la revendication 7 ou 8 ou élément de prélèvement d'échantillon et d'analyse intégré jetable selon l'une des revendications 1 à 6, **caractérisé en ce que** la zone de réception d'échantillon (10) comporte une zone de test (38) qui comprend au moins une couche absorbante et qui présente une surface de réception d'échantillon (40) sur le côté (11) en contact avec l'échantillon de la bandelette de test (6) et une surface de détection (41) sur le côté opposé audit côté (11) en contact avec l'échantillon de la bandelette de test (6).

10. Système d'analyse selon l'une des revendications 7 à 9 ou élément de prélèvement d'échantillon et d'analyse intégré jetable selon l'une des revendications 1 à 6, **caractérisé en ce que** la pointe (15) de l'élément piquant (7) est amenée pendant la phase de propulsion du mouvement de piqûre au-delà de l'extrémité avant (30) de la bandelette de test (6) dans le sens de la piqûre.

11. Système d'analyse selon l'une des revendications 7 à 10, **caractérisé en ce que** le mouvement de piqûre de l'élément piquant (7) est suivi d'un mouvement de collecte au cours duquel un mouvement relatif plus lent que le mouvement de piqûre ou bien aucun mouvement relatif de l'élément piquant (7) par rapport à la bandelette de test (6) n'est effectué.

12. Système d'analyse selon l'une des revendications 10 et 11, **caractérisé en ce que** pendant le mouvement de collecte, un échantillon de liquide corporel est reçu dans le canal capillaire (17) de l'élément piquant (7).

13. Système d'analyse selon l'une des revendications 7 à 12, **caractérisé en ce que** l'appareil d'analyse (2) comporte un dispositif d'application (34), permettant d'appliquer l'élément piquant (7) en position de transfert d'échantillon sur la zone de réception d'échantillon (10) afin de transférer le liquide corporel vers ladite zone de réception d'échantillon (10).

14. Système d'analyse selon l'une des revendications 7 à 13, **caractérisé en ce que** l'extrémité avant (30) de la bandelette de test (3) dans le sens de la piqûre présente une surface (31) en contact avec la peau et sert d'élément de référence (33) pour la profondeur de piqûre, sachant qu'une valeur prédéfinie de la profondeur de piqûre est déterminée par la distance dans le sens de la piqûre entre la surface (31) en contact avec la peau et la position de la pointe (15) de l'élément piquant (7) au point de retour du mouvement de piqûre.

15. Système d'analyse selon la revendication 14, **caractérisé en ce qu'**une unité de raccourcissement est intégrée dans le système d'analyse (1) en vue de raccourcir la bandelette de test (6) à son extrémité avant (30) dans le sens de la piqûre.

16. Système d'analyse selon l'une des revendications 7 à 15, **caractérisé en ce que** l'unité de couplage comprend un premier mécanisme de couplage (25) pour le couplage avec l'élément piquant (7) et un second mécanisme de couplage (27) pour le couplage avec la bandelette de test (6).

17. Système d'analyse selon l'une des revendications 7 à 16, **caractérisé en ce que** plusieurs éléments de prélèvement d'échantillon et d'analyse sont stockés dans un magasin d'empilage et peuvent être retirés un à un du magasin d'empilage et être amenés dans une position de couplage.
